(19) Europäisches Patentamt
European Patent Office
Office européen des brevets

(11) **EP 3 848 705 B1**

(12) **EUROPEAN PATENT SPECIFICATION**

(45) Date of publication and mention
of the grant of the patent:
**17.05.2023 Bulletin 2023/20**

(21) Application number: **21150966.6**

(22) Date of filing: **11.01.2021**

(51) International Patent Classification (IPC):
**G01N 33/483** (2006.01)     **B01L 3/00** (2006.01)
**C12N 5/077** (2010.01)      **G01N 33/50** (2006.01)
**C12N 5/071** (2010.01)

(52) Cooperative Patent Classification (CPC):
**G01N 33/4833; B01L 3/5085; C12N 5/0657;
G01N 33/5014; G01N 33/5061; G01N 33/5073;**
B01L 2200/12; B01L 2300/0663; B01L 2300/123;
B01L 2300/163; C12N 5/0697; C12N 2500/02;
C12N 2500/38; C12N 2501/15; C12N 2501/155;

(Cont.)

(54) **MICROFABRICATED DEVICES AND HIGH THROUGHPUT ASSAYS FOR MODULATORS OF CELL BEHAVIOR**

MIKROHERGESTELLTE VORRICHTUNGEN UND HOCHDURCHSATZASSAYS FÜR MODULATOREN DES ZELLVERHALTENS

DISPOSITIFS MICROFABRIQUÉS ET ESSAIS À HAUT DÉBIT POUR DES MODULATEURS DU COMPORTEMENT CELLULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.01.2020 US 202062959040 P**

(43) Date of publication of application:
**14.07.2021 Bulletin 2021/28**

(73) Proprietor: **Novoheart Limited
Kowloon (HK)**

(72) Inventors:
• **TRAN, David D.
Aliso Viejo, CA 92656 (US)**
• **LEE, Eugene K.
Kwun Tong, Kowloon (HK)**
• **KUROKAWA, Yosuke
Kwun Tong, Kowloon (HK)**
• **ROBERTS, Erin G.
Kwun Tong, Kowloon (HK)**
• **COSTA, Kevin D.
NY 10026 (US)**

(74) Representative: **Grünecker Patent- und
Rechtsanwälte
PartG mbB
Leopoldstraße 4
80802 München (DE)**

(56) References cited:
**WO-A1-2019/244044    US-A1- 2019 203 179**

• **ARNE HANSEN ET AL: "Development of a drug screening platform based on engineered heart tissue", CIRCULATION RESEARCH, GRUNE AND STRATTON, BALTIMORE, US, vol. 107, no. 1, 9 July 2010 (2010-07-09), page 35, XP002671600, ISSN: 0009-7330, DOI: 10.1161/CIRCRESAHA.109.211458 [retrieved on 2010-05-06]**
• **INGRA VOLLERT ET AL: "In-vitro perfusion of engineered heart tissue through endothelialized channels", TISSUE ENGINEERING PART A, 25 October 2013 (2013-10-25), XP055506701, US ISSN: 1937-3341, DOI: 10.1089/ten.TEA.2013.0214**

(52) Cooperative Patent Classification (CPC): (Cont.)
C12N 2501/415; C12N 2501/727; C12N 2502/09;
C12N 2502/1329; C12N 2506/02; C12N 2513/00;
C12N 2533/54; C12N 2533/90

## Description

### Field

The invention relates to the field of microfabrication and high throughput methods screening for modulators of cellular behavior.

### Background

Cardiotoxicity remains a critical challenge in the development of new drugs, leading to significant drug attrition during Phase I-III clinical trials and after market approval [1]. This failure is partly attributed to the lack of an effective preclinical drug screening platform. The current standard for preclinical screening utilizes animal models, which have proven to be inadequate in predicting cardiotoxicity due to interspecies differences in the cardiac physiology compared to humans [2]. To this end, researchers have focused significant efforts in using cardiomyocytes (CMs) derived from human pluripotent stem cells (hPSCs), including induced pluripotent stem cells (iPSCs) reprogrammed from differentiated cells, as a preclinical drug screening tool that can recapitulate the physiological human response to cardioactive compounds [3,4],

Several drug screening platforms have been recently developed using hPSC-derived CMs (hPSC-CMs) [5]. One such platform takes the form of a 3-dimensional cardiac construct that is anchored at two points in order to create an aligned, elongated tissue that generates uniaxial force. Such a tissue platform, termed a tissue strip or TS (e.g., a cardiac tissue strip (CTS)), have been used to validate physiological responses to a wide range of drugs, including some that have failed traditional preclinical screening methods [6].

While the high-content nature of the TS platforms shows strong promise for use in drug development and drug screening applications, most TS platforms are limited by their lower throughput compared to simpler platforms. High-throughput screening is a part of standard pharmaceutical preclinical drug development pipelines, involving several rounds of screening to identify and optimize candidate drugs [7]. In order to address this need, a high-throughput TS platform that is compatible with industry-standard screening tools is needed.

Other TS platforms are known from ARNE HANSEN ET AL: "Development of a drug screening platform based on engineered heart tissue",CIRCULATION RESEARCH, GRUNE AND STRATTON, BALTIMORE, US, vol. 107, no. 1, 9 July 2010 (2010-07-09), page 35, XP002671600,ISSN: 0009-7330, DOI: 10.1161/CIRCRESAHA.109.211458 and INGRA VOLLERT ET AL: "In-vitro perfusion of engineered heart tissue through endothelialized channels",TISSUE ENGINEERING PART A, 25 October 2013 (2013-10-25), XP055506701,US ISSN: 1937-3341, DOI: 10.1089/ten.TEA.2013.0214.

## Summary

The invention provides microfabricated devices, systems incorporating such devices and methods for using such devices to screen modulators, e.g., drugs, of cell or tissue behavior. The microfabricated device comprises two posts, or anchors, to which a tissue strip attaches. The anchors are flexible to allow movement of the tissue strip, which allows measurement of any change in force exerted by the tissue strip in relation to changes in conditions. The microfabricated devices are amenable to incorporation in high-throughput assay formats. For example, high-throughput assays using the microfabricated devices can be formatted for 96-well clear polystyrene plate formats that are suitable for automated measurements and monitoring using plate readers known in the art. In addition to compatibility with 96-well plate formats, the microfabricated devices can be formatted for 6-well, 12-well, 24-well, and 48-well plates. In some cases, plates are made of opaque material for use in fluorescent assays. Furthermore in addition to measurements, standard plate formatting is important for adaptation to other automated machine systems such as media exchange, reagent addition, and mixing. The microfabricated devices are also compatible with electrical stimulation of tissue strips attached to the anchors. In some embodiments, a trough is provided for seeding that allows electrical stimulation by concentrating seeding in specific location due to surface tension. The microfabricated devices disclosed herein provide an advantage in both force output and electrophysiology over designs characterized by tissue rings, which have been previously described (WO 2014/085933). Rings may have uncoordinated/asynchronous force contraction within the tissue, reducing signal in the measurement system. Also, ring systems may exhibit a circular electrical wave, leading to uncoordinated contraction of cells capable of coordinated contraction, such as myocytes. Further, ring-based systems may have multiple simultaneous electrical waves. It is therefore desirable to design linear tissues amenable to high-throughput measurement to overcome these shortcomings of tissue rings.

Described herein are various designs of a tissue strip (TS) platform compatible with standard cell culture plates for use in high-throughput applications, including automated robotic multi-well plate handling systems. In particular, the tissue strip platform is suitable for the development and manipulation of a cardiac tissue strip (CTS), although a variety of tissue strips are contemplated by the invention, including visceral organ tissue strips such as liver, stomach, pancreas, gall bladder, kidney, small intestine, colon, urethra, ureter, bladder, prostate, uterus, and ovary tissue strips as well as eye, skin, brain, tongue, esophagus, vascular, tendon, ligament, skeletal muscle and smooth muscle tissue strips.

In one aspect, the invention provides a microfabricated device comprising an intact and functional miniature tissue for high-throughput screening of modulators of biological tissue activity comprising at least two biocompatible posts and a substrate to which the posts are attached, wherein the distance separating at least two posts is at least 0.5 mm, and wherein each post comprises an elastomeric material, a force sensor, and a feature for tethering a tissue strip wherein the feature for tethering a tissue strip is a curve in the post, wherein the tissue strip comprises a composition comprising cells of at least one force-generating cell type and one type of extracellular matrix, further wherein the microfabricated device is suitable for monitoring tissue strip position over time, *in situ.* In some embodiments, the suitability for monitoring tissue strip position over time is further suitable for providing a measurement of tissue strip movement *in situ.* In some embodiments, the force-generating cell type is a smooth muscle cell type, a skeletal muscle cell type, a cardiac muscle cell type, a fibroblast cell type, a neutrophil cell type, an eosinophil cell type, a basophil cell type, a monocyte cell type, a lymphocyte cell type, a glial cell type, a chondrocyte cell type, an osteoblast cell type, an osteoclast cell type, an osteocyte cell type, a keratinocyte cell type, a melanocyte cell type, a Merkel cell type, a dendritic cell type, an endothelial cell type, an epithelial cell type, a white adipocyte cell type, a brown adipocyte cell type, an esophageal cell type, a pharynx cell type, a larynx cell type, a lung cell type, an hepatocyte cell type, a bladder cell type, a kidney cell type, a stomach cell type, a gallbladder cell type, beta islet cell type, a spleen cell type, a small intestine cell type, or a colon cell type. In some embodiments, the force-generating cell type is a stem cell-derived cell type, such as a myocyte cell type. In some embodiments, the myocyte cell type is a cardiomyocyte cell type, which may be a primate, horse, cow, goat, sheep, zebrafish, pig, dog, rat, mouse, or a human cardiomyocyte cell type, *e.g.,* a human ventricular cardiomyocyte cell type.

In some embodiments, the elastomeric material is a polymer, such as polydimethylsiloxane, polyurethane, polyethylene, or polyacrylamide. In some embodiments, the substrate is thin-layer silicone, such as thin-layer polydimethylsiloxane, thin-layer polyurethane, thin-layer polyethylene, or thin-layer polyacrylamide.

In some embodiments, the microfabricated device further comprises a recording device to monitor tissue strip position or to detect movement of a tissue strip. In some embodiments, the microfabricated device further comprises two unipolar electrodes for field stimulation, a bipolar micro-electrode for point contact stimulation, or a micro-cannula for contacting a tissue strip with an electrical stimulus or a modulator of a biological tissue activity.

Another aspect of the invention is drawn to a system for measuring tissue activity comprising: (a) a microfabricated device as disclosed herein; and (b) a recording device for capturing the force detected by at least one force sensor of the microfabricated device. In some embodiments, the tissue comprises a cell type that is a smooth muscle cell type, a skeletal muscle cell type, a cardiac muscle cell type, a fibroblast cell type, a neutrophil cell type, an eosinophil cell type, a basophil cell type, a monocyte cell type, a lymphocyte cell type, a glial cell type, a chondrocyte cell type, an osteoblast cell type, an osteoclast cell type, an osteocyte cell type, a keratinocyte cell type, a melanocyte cell type, a Merkel cell type, a dendritic cell type, an endothelial cell type, an epithelial cell type, a white adipocyte cell type, a brown adipocyte cell type, an esophageal cell type, a pharynx cell type, a larynx cell type, a lung cell type, an hepatocyte cell type, a bladder cell type, a kidney cell type, a stomach cell type, a gallbladder cell type, beta islet cell type, a spleen cell type, a small intestine cell type, or a colon cell type. In some embodiments, the tissue comprises a stem cell-derived cell type. In some embodiments, the tissue comprises a myocyte cell type, such as a cardiomyocyte cell type, which may be a primate, horse, cow, goat, sheep, zebrafish, pig, dog, rat, mouse, or a human cardiomyocyte cell type, *e.g.*, a human ventricular cardiomyocyte cell type. In some embodiments, the system further comprises two unipolar electrodes for field stimulation, a bipolar micro-electrode for point contact stimulation, or a micro-cannula for contacting a tissue composition comprising the cells of at least one cell type with an electrical stimulus or a modulator of a biological tissue activity.

Yet another aspect of the invention is a method for assaying a property of a tissue strip comprising: (a) exposing the tissue strip in a microfabricated device as disclosed herein to an electrical stimulus or a modulator of a biological activity; and (b) measuring the response of the tissue strip, wherein the response of the tissue strip is compared to a control or baseline measurement of a tissue strip of the same cell type or types not exposed to the electrical stimulus or modulator of a biological activity. In some embodiments, the tissue comprises a cell type that is a smooth muscle cell type, a skeletal muscle cell type, a cardiac muscle cell type, a fibroblast cell type, a neutrophil cell type, an eosinophil cell type, a basophil cell type, a monocyte cell type, a lymphocyte cell type, a glial cell type, a chondrocyte cell type, an osteoblast cell type, an osteoclast cell type, an osteocyte cell type, a keratinocyte cell type, a melanocyte cell type, a Merkel cell type, a dendritic cell type, an endothelial cell type, an epithelial cell type, a white adipocyte cell type, a brown adipocyte cell type, an esophageal cell type, a pharynx cell type, a larynx cell type, a lung cell type, an hepatocyte cell type, a bladder cell type, a kidney cell type, a stomach cell type, a gallbladder cell type, beta islet cell type, a spleen cell type, a small intestine cell type, or a colon cell type. In some embodiments, the tissue comprises a stem cell-derived cell type. In some embodiments, the tissue comprises a myocyte cell type, such as a cardiomyocyte cell type, which may be any mammalian cardiomyocyte cell type, including a primate, horse, cow, goat, sheep, zebrafish, pig, dog, rat, mouse, or a human cardiomyocyte cell type, *e.g.*, a human ventricular cardiomyocyte cell type. In some embodiments of the method, the response is contractile force. In some embodiments, the microfabricated device further comprises a recording device that detects the presence or absence of movement of the tissue strip, wherein movement of the tissue strip results from a change in contractile force.

Other features and advantages of the disclosure will become apparent from the following detailed description, including the drawings. It should be understood, however, that the detailed description and the specific examples, while indicating embodiments, are provided for illustration only.

**Brief Description of the Drawings**

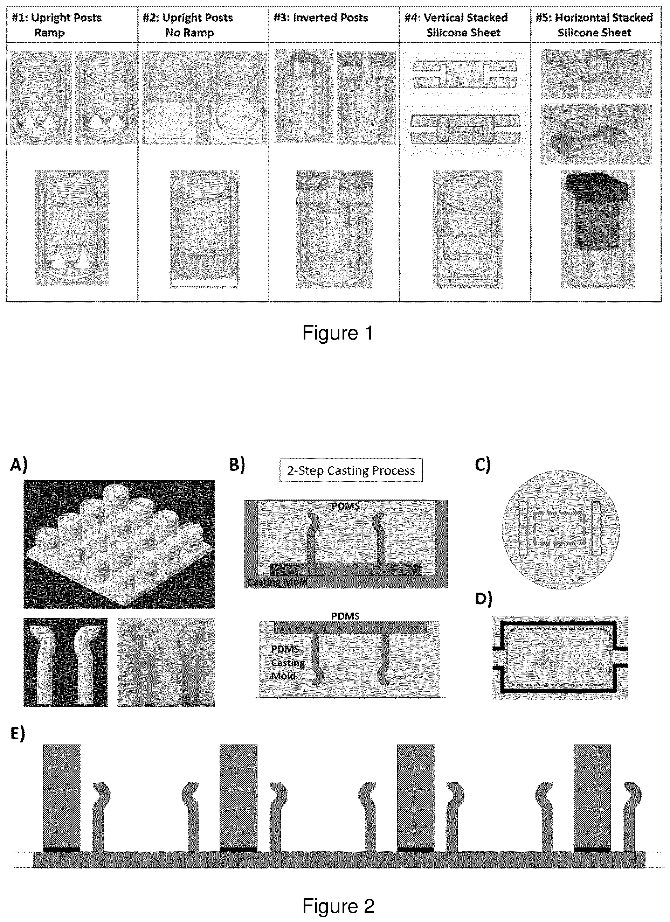

Figure 1. Designs of high-throughput TS platform

Figure 2. Fabrication and design of silicone insert for Design Number 1 and 2 (Figure 1). A) Design of 4x4 array of inserts (top) and curved posts (bottom), showing side-by-side views of designed curved posts (left) and 3D-printed curved posts (right). The 3D-printed posts were generated using stereo lithography (SLA), a process for creating 3D objects in which a computer-controlled moving laser beam is used to build up the required structure, layer by layer, from a liquid polymer that hardens on contact with laser light. B) The 2-Step casting process consists of using a resin SLA-printed positive casting mold to cast a silicone negative mold (top), which is then used to cast the final positive silicone insert (bottom). C) Top-down view of insert. Dashed boundary line denotes the central tissue seeding area with posts. Solid boundary lines denote areas for electrode insertion. D) Close-up of central tissue seeding area and posts. Dashed boundary line denotes boundary of tissue seeding mixture. Channels on the outside of the tissue seeding area lead to the electrode areas to permit electrical stimulation. The channels are designed in such a way that the surface tension of the tissue seeding mixture prevents the liquid from leaking out of the seeding area into the surrounding channels. E) Side-profile view of fabricated plate. Silicone insert (dark gray) is adhered to a 96-well bottomless plate (crosshatch) using a thin layer of silicone-based adhesive (black).

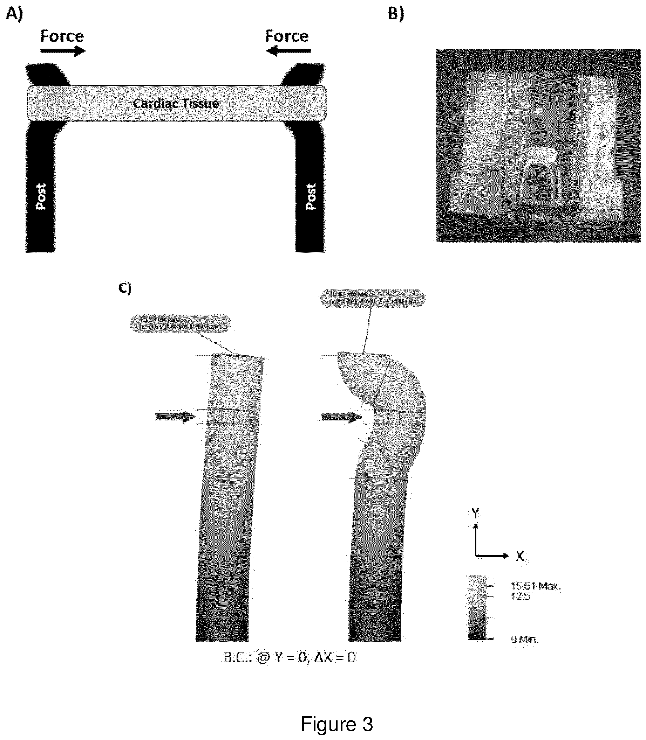

Figure 3. Deflection of tissue connected to posts. A) Tissue formation attached to the curved posts. Post is designed with a curved feature intended to retain the tissue at a specific height. As the tissue contracts and exerts force inwards, the post would deflect inwards and the distance of deflection is measured. B) Example side profile image of tissue compaction around posts. C) Finite element modeling of a straight post versus a post with curvature. Boundary conditions for the post assumes the post is fixed at the bottom (Y = 0) with no x-displacement ($\Delta$X = 0).

For an applied 10 $\mu$N load, the deflection of both posts are similar. Therefore, classical beam bending theory may be applied to derive force of a cardiac tissue from displacement of the top of the curved post. Gray scale represents x-displacement, ranging from 0 $\mu$m (black) to 15 $\mu$m (light gray).

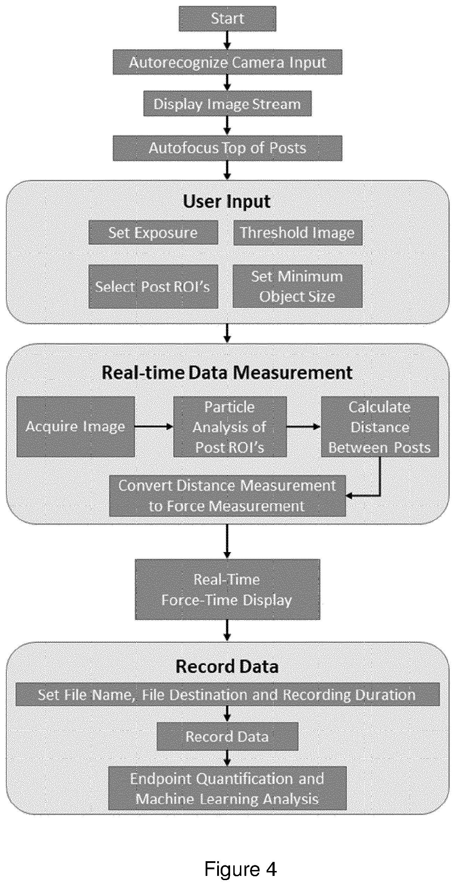

Figure 4. Flow diagrams of LabVIEW software to measure displacement of posts and derive force of tissue contraction. The flow diagram schematics include camera control, user input for setup of the system, real-time data measurement of post displacement and force, and data recording features. Recorded data can then be exported for quantification and analysis using machine learning.

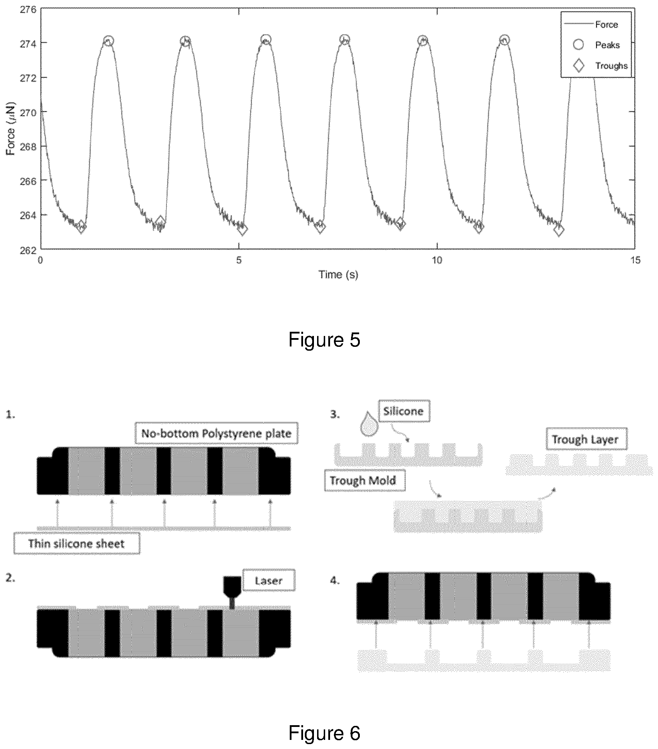

Figure 5. Exemplary twitch force tracing of cardiac microtissue from Design Number 2 (see Figure 1). A custom MATLAB analysis code was used to automatically detect the local peak ('circle') and trough ('diamond') for each cycle of the force tracings.

Figure 6. Fabrication overview of stacked tissue culture plate for Design Number 4 (see Figure 1). 1) Bonding of thin silicone sheet to bottomless polystyrene plate (black). 2) Turn plate upside down and laser cut thin silicone sheet to specific patterns. 3) Casting a silicone trough in the form of a polydimethylsiloxane (PDMS) trough from trough mold (gray). 4) Adhering of trough layer, from step 3, to bottomless plate with thin patterned silicone sheet, from step 2.

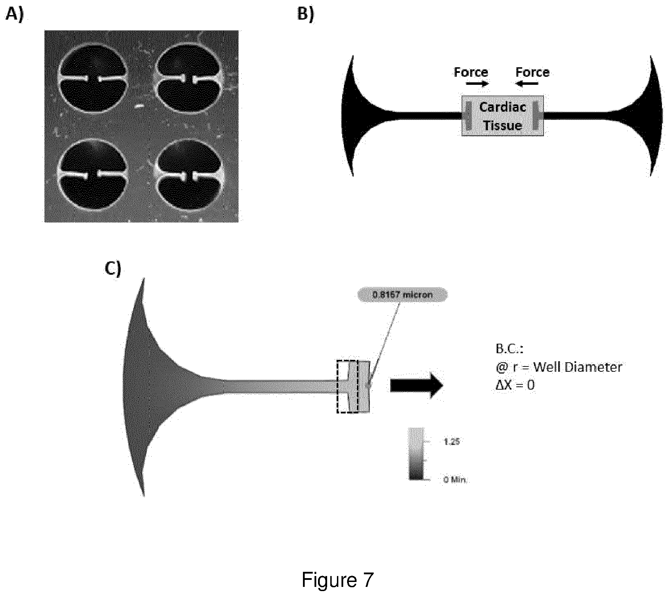

Figure 7. Laser-cut thin silicone sheets for fabrication of Design Number 4 (see Figure 1). A) Example laser-cut attachment arms of varying dimensions. A 'fillet' feature at the base improved stability and alignment of the arms. B) Tissue formation (gray) attached to the laser-cut silicone arms (black). As the tissue contracts and exerts force inwards, the silicone arms stretch inwards and the distance of stretch is measured. C) Example finite element modeling of tissue attachment to thin silicone 'arm', assuming silicone sheet thickness of 300 $\mu$m and 10 $\mu$N force applied to each arm by tissue. Boundary conditions for the silicone arm assumes the arm is fixed to the edge of the well (radius = Well Diameter) with no x-displacement ($\Delta$X = 0). Black dashed outlines illustrate the relaxed state with no force applied. Gray scale represents displacement from relaxed state to contracted state (10 $\mu$N force), ranging from 0 $\mu$m (black) to 1.25 $\mu$m (light gray).

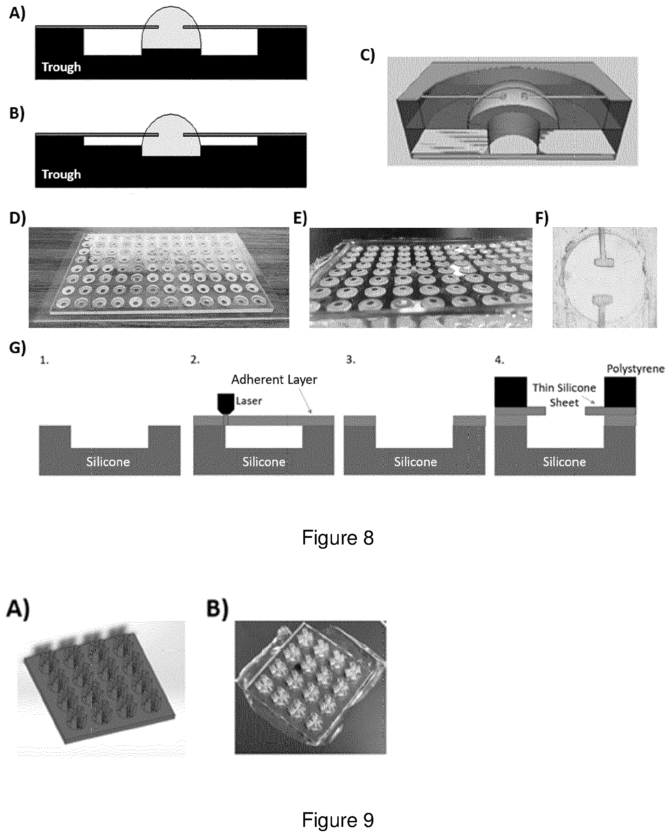

Figure 8. Troughs for stacked tissue culture plates. A) Example of trough (black) design with pedestal. Surface tension of tissue seeding mixture (light gray) and the hydrophobicity of trough keep the mixture in a specific position for confining the cell mixture around the attachment points of silicone 'arms' (dark gray). B) Example of trough (black) design with recessed area for tissue seeding. C) Isometric cutaway of stacked tissue culture plate with recessed trough. D) Laser-cut casting mold made from acrylic. E) Silicone (in the form of PDMS) casting mold. F) Example top-down view of single well with silicone 'arms' and attached trough with recessed area for tissue seeding. G) Attachment of silicone (PDMS) trough layer to thin silicone sheet and polystyrene culture plate with laser-cut adherent layer.

**Figure 9.** A) CAD model of trough insert. B) Example of double-casted 4x4 PDMS trough.

## Detailed Description

The invention provides microfabricated devices suitable for generating tissue strips useful in assessing the function of cells organized in tissue-like environments as well as methods for assessing the behavior of such tissue strips and methods of screening for modulators of the function(s) of the tissues. Any plate format suitable for high throughput assays is suitable for use in a microfabricated device according to the invention. Exemplary high-throughput assays can be formatted for 96-well clear polystyrene plate formats that are suitable for automated measurements and monitoring using plate readers known in the art. Six-well, 12-well, 24-well, and 48-well plate formats are plates suitable for incorporation into the microfabricated devices of the invention. In some cases, plates are made of opaque material to be amenable to fluorescent assays. Furthermore, in addition to measurements, standard plate formatting is important for adaptation to other automated machine systems, such as media exchange, reagent addition, mixing, and the like.

The curved post geometry and the scale required to make such small tissues meant that straightforward micromachining of the master casting mold would not be an option. Therefore, we had to turn to stereolithography or 3D printing to produce the master mold. 3D printing the curved geometry at this scale was still a challenge due to the resolution limitations of current 3D printers. Moreover, the curved feature itself was a challenge to print due to the overhanging geometry and the scale. A variety of resins were tested to find the current design. Resins that performed well and are expected to be useful include Accura ClearVue (3D Systems), Black Resin (Formlabs). Resins that didn't work are: VisiJet FTX Green (3D Systems), Clear Resin (Formlabs), and Grey Resin (Formlabs). In addition, casting the PDMS post was challenging due to the geometry of the posts. We could not simply perform a single elastomeric (*e.g.*, PDMS) casting, but rather had to adopt a two-step casting process that consisted of: (a) printing a positive master mold using a 3D printer, then (b) casting a soft negative mold (*e.g.*, PDMS) from the positive mold, and then c) casting the final part. For both straight posts and curved posts, we are able to modify the dimensions of the posts, such as diameter and height, to adjust and detect a specific range of forces. The dimensions of the posts will be designed in order for the posts to move a minimum distance as detectable by an imaging system. In some cases, the detection limit may be one camera pixel which translates to a minimum detection distance of 10 micrometers.

The main advantage of curved posts is that the curved feature retains the tissue at a specific position. Without the feature, a tissue may form and attach to any point on the post, and in some cases, the tissue may rise up the post and ultimately detach from the post. Furthermore, it is very important to know the height at which the tissue attaches to the post as the height is used in the beam-bending equation to derive the force of the cardiac tissue from the displacement of the posts. In more macroscopic formats (such as 6-well plate formats), it is feasible to image the tissue from the side to confirm the attachment point in order to accurately derive the force. In a 96-well plate format, however, it is difficult and time-consuming to measure the height of the tissues upon the posts. Thus, curved posts provide the advantage of physically retaining the tissues at a certain height, which facilitates long-term stability of the tissues and accuracy of derived force measurements.

Some of the microfabricated device designs have inverted posts. One advantage shared by the inverted designs is the minimization of material between the bottom of the plate and the tip of the posts when using inverted microscopy, a common high-throughput automated imaging modality. This allows direct tracking of the post tips and minimizes image distortion. The difficulties in engineering tissue strips using the inverted designs is the limited space above the 96-well plate that must hold the posts for tissue attachment and provide room for additional features necessary for tissue culturing (e.g., media feeding, aspiration), imaging, and in certain cases (*e.g.*, cardiac tissues) electrical stimulation.

Suitable parameters for a microfabricated device according to the invention that comprises a plate with 96 wells and is compatible with high-throughput assaying for modulators of cell or tissue behavior include the following. Post dimensions of 0.5 - 2 mm in diameter and 0.5 - 6 mm in height. Distance between posts may be up to 6 mm apart. Materials suitable for use in making the posts include elastomers such as thermoplastics and silicones, e.g., PDMS. Generally, any material that is biocompatible and soft enough to deform when undergoing low forces of about 5-500 micronewtons is suitable. An exemplary material is PDMS, which has a Young's modulus of 1.32-2.97 MPa.

Further challenges that had to be overcome were challenges with fabricating the plates, including the adhesion of multiple layers of material to the bottom of the plates to prevent leaking, special design considerations to allow electrical stimulation, careful examination of post curvature design to ensure effective tissue trap and fabrication feasibility, and optimization of sensor geometry for force sensitivity. Regarding electrical stimulation, the electrode area is designed to be large enough for ease of electrode insertion by the operator. The channels between the electrode area and the center seeding area are wider than the pole diameter to maximize the effective electric field upon the tissue. Another factor affecting the design is that the channel should not be too wide because the tissue seeding mixture is held in the tissue seeding area by surface tension, which relies on a small channel width.

Regarding post curvature, we determined the maximum curvature at which printing was feasibly successful. After fabrication, tissues were grown to confirm that tissues were retained on posts with higher curvature for longer periods of culture time.

Regarding adhesion of material to the bottom of plates, in Design 2, the adhesive layer must be viscous enough to prevent air gaps from forming after pressing the insert to the plate. Air gaps in the adherent layer result in leakage when using the plate.

Regarding optimization of force sensitivity in the multilayered fabrication method, there is difficulty in lasering very thin materials as too high of laser power and laser density can cause burning of the thin layer, resulting in curling of the material at resting state.

The invention also provides for monitoring TS behavior using a recording device. Any device for recording tissue motion is suitable for use with microfabricated devices, including any camera, including any video camera, or camcorder known in the art. Further, the recording device may be any electrical or electromechanical device for recording the force detected by a force sensor of a post in a microfabricated device according to the invention.

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs.

The term "comprising" or "comprises" is used in reference to manufactures, compositions, methods, and respective component(s) thereof, that are essential to the method, manufacture, or composition, yet open to the inclusion of unspecified elements, whether essential or not.

The term "consisting essentially of" refers to those elements required for a given embodiment. The term permits the presence of elements that do not materially affect the basic and novel characteristic(s) of that embodiment.

The term "consisting of" refers to compositions, methods, and respective components thereof as described herein, which are exclusive of any element not recited in that description of the embodiment.

As used in this specification and the appended claims, the singular forms "a", "an", and "the" include plural references unless the context indicates otherwise. For example, references to "the method" include one or more methods, and/or steps of the type described herein and/or which will become apparent to those of skill in the art upon reading this disclosure. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is used herein to indicate a non-limiting example. Thus, the abbreviation "e.g." is synonymous with the term "for example."

Definitions of common terms in cell biology and molecular biology can be found in "the Merck Manual of Diagnosis and Therapy", 19th edition, published by Merck Research Laboratories, 2006 (ISBN 0-911910-19-0); Robert S. Porter et al. (eds.), The Encyclopedia of Molecular Biology, published by Blackwell Science Ltd., 1994 (ISBN 0-632-02182-9); the ELISA Guidebook (Methods in Molecular Biology 149) by Crowther J. R. (2000). Definitions of common terms in molecular biology can also be found in Benjamin Lewin, Genes X, published by Jones & Bartlett Publishing, 2009 (ISBN-10: 0763766321); Kendrew et al. (eds.), Molecular Biology and Biotechnology: A Comprehensive Desk Reference, published by VCH Publishers, Inc., 1995 (ISBN 1-56081-569-8).

Unless otherwise indicated, the methods disclosed herein were performed using standard procedures, as described, e.g., in Sambrook et al., Molecular Cloning: A Laboratory Manual (3 ed.), Cold Spring Harbor Laboratory Press, Cold Spring Harbor, N.U., USA (2001); Davis et al., Basic Methods in Molecular Biology, Elsevier Science Publishing, Inc., New York, USA (1995); Current Protocols in Cell Biology (CPCB) (Juan S. Bonifacino et. al. ed., John Wiley and Sons, Inc.); Culture of Animal Cells: A Manual of Basic Technique by R. Ian Freshney, publisher: Wiley-Liss; 5th edition (2005); and Animal Cell Culture Methods (Methods in Cell Biology, vol. 57, Jennie P. Mather and David Barnes editors, academic press, 1st edition, 1998).

The terms "inhibit", "decrease," "reduce," "reduced", and "reduction" are all used herein generally to mean a decrease by a statistically significant amount relative to a reference. To avoid all doubt, however, "inhibit", "reduce," "reduction", or "decrease" typically means a decrease by at least 10% as compared to the absence of a given treatment and can include, for example, a decrease by at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 98%, at least 99%, up to and including, for example, the complete absence of the given entity or parameter as compared to the absence of a given treatment, or any decrease between 10-99% as compared to the absence of a given treatment.

The terms "stimulated", "increased", "increase", or "enhance" are all used herein to generally mean an increase by a statistically significant amount; to avoid doubt, the terms "stimulated", "increased", "increase", or "enhance" means an increase of at least 10% as compared to a reference level, for example an increase of at least 20%, or at least 30%, or at least 40%, or at least 50%, or at least 60%, or at least 70%, or at least 80%, or at least 90% or up to and including a 100% increase or any increase between 10-100% as compared to a reference level, or at least a 2-fold, or at least a 3-fold, or at least a 4-fold, or at least a 5-fold or at least a 10-fold increase, or any increase between 2-fold and 10-fold or greater as compared to a reference level.

As used herein, "maintaining" or "culturing" refers to continuing the viability of a tissue or population of cells. A maintained tissue will have a population of metabolically active cells. The number of these cells can be roughly stable over a period of at least 3 days or can grow.

The term "microfabricated device" refers to a structure or substrate constructed on a small scale compatible with materials and supplies known to be useful in high-throughput assays, such as 96-well plates.

As used herein, the terms "induced pluripotent stem cell" or "iPSC", which are used interchangeably herein, refer to pluripotent cells derived from differentiated cells. For example, iPSCs can be obtained by overexpression of transcription factors such as Oct4, Sox2, c-Myc and Klf4 according to the methods described in Takahashi et al. (Cell, 126: 663-676, 2006). Other methods for producing iPSCs are described, for example, in Takahashi et al. Cell, 131: 861-872, 2007 and Nakagawa et al. Nat. Biotechnol. 26: 101-106, 2008. In some embodiments, iPSCs are cultured in the presence of microfabricated devices under conditions that lead to the development of tissue strips according to the disclosure, such as human ventricular cardiac tissue strips (hvCTSs).

The term "statistically significant" or "significantly" refers to statistical significance and generally means a measurement that is two standard deviations (2sd) different from its basis of comparison. The term refers to statistical evidence that there is a difference. It is defined as the probability of making a decision to reject the null hypothesis when the null hypothesis is actually true. The decision is often made using the p-value.

Other than in the operating examples, or where otherwise indicated, all numbers expressing quantities of ingredients or reaction conditions used herein should be understood as modified in all instances by the term "about." The term "about", when used in connection with percentages, can mean plus or minus 1%.

The following examples illustrate the subject matter of the disclosure and are not intended to limit the scope of the disclosure. Example 1 provides the methods used in the experiments disclosed herein, Example 2 describes experiments measuring the developed force generated by TS, e.g., CTS, using curved posts, Example 3 describes the fabrication and assembly of vertical stacked cardiac tissue strip culture plates, Example 4 describes the bonding of thin silicone sheets to bottomless cell culture plates in generating some embodiments of microfabricated devices according to the invention, Example 5 discusses the cutting of thin silicone sheet with a laser to prepare customized materials for fabricated devices according to the invention, and Example 6 describes trough layer fabrication for use in preparing the microfabricated devices according to the invention and tissue strips according to the disclosure

## Examples

### *Example 1*

Methods

#### *Cardiomyocyte Preparation*

Human embryonic stem cells (hESC), using the hES2 cell line, were differentiated into human ventricular-like cardiomyocytes (hvCMs) following Novoheart's proprietary protocol based on the embryoid body method [8]. In brief, cells were maintained on Matrigel coated plates with mTeSR1 at 37°C with 5% $CO_2$. On the first day of differentiation, cells were digested to form small cell clusters suspended in mTeSR1 with Matrigel, 1 ng/ml bone morphogenetic protein 4 (BMP4) and 10 $\mu$M ROCK inhibitor Y-27632 (RI) for 24 hours in an ultra-low attachment plate under hypoxic conditions. Medium was then replaced with StemPro-34 medium with GlutaMAX supplemented with 50 $\mu$g/mL ascorbic acid, 10 ng/mL activin A, 10 ng/mL BMP4, and 5 $\mu$M RI. After 3 days, cells were cultured in StemPro-34 medium supplemented with 50 $\mu$g/mL ascorbic acid and 5 $\mu$M IWR-1 for 4 days. Afterwards, cell clusters were maintained in RPMI 1640 supplemented with B27 and 50 $\mu$g/mL ascorbic acid in normoxic condition until the day of tissue fabrication. Batches were assessed with flow cytometry on differentiation day 13 or 14 for cardiac troponin T-positive cells, with a quality control criterion of at least 60% cTnT+ cells.

#### *Generation of Cardiac Microtissues*

hvCMs were dissociated into single cells on differentiation day 15 and allowed to recover for 72 hours before the fabrication of miniature cardiac tissues. Prior to cell seeding, the 96-well prototype plate was sterilized with ethanol and air-dried under UV for 15 minutes before coating with 2% BSA at 37°C for one hour. After coating, hvCMs were mixed with human foreskin fibroblasts in 10:1 ratio and suspended in ice-cold solution of collagen mixture. The final cell-collagen mixture was added to the well and allowed to gel in the incubator. After 1 hour, DMEM medium supplemented with 10% newborn calf serum was added. Medium was changed every other day until the day of measurement.

*Potential Variations of a High-throughput TS platform*

A high-throughput TS platform (*e.g.*, a CTS platform) can be fabricated in multiple different configurations (Figure 1). Each configuration is designed for the following: (1) 96-well format, (2) two anchor points to create aligned, elongated tissue, (3) one tissue strip per well, (4) in-well optical monitoring of contractile force, (5) in-well electrical pacing compatibility, and (6) design elements for standardization of tissue z-height. In some embodiments, the platform can be scaled to other standard multi-well formats, such as, but not limited to, 48-well, 24-well, 12-well, 6-well formats, and the like.

All design configurations included silicone inserts fitted to commercially available polystyrene plates. Design numbers 1, 2 and 4 present silicone-based features attached to the bottom of a bottomless 96-well polystyrene plate, while Design numbers 3 and 5 present PDMS-based features inserted from above into a standard 96-well polystyrene plate. The designs also include anchor points designed to displace a detectable distance in order to optically derive force measurements. In some embodiments, the inserts may be fabricated out of other biocompatible soft materials, such as, but not limited to, polydimethylsiloxane (PDMS), other synthetic polymers, natural occurring polymers, or other biomaterials. In some embodiments, the plate may be fabricated in different material than polystyrene, such as, but not limited to, polymethylmetacrylate (PMMA), and polycarbonate (PC).

Design Number 1 features two uprights posts for tissue attachment, with each post residing on top of its own individual ramp. The posts include a feature at the tip of the post to prevent tissues from migrating up the posts and detaching from the system, or from sliding down the posts to an unspecified z-height. The ramp is designed for the tissue seeding mixture to be injected at the bottom of the ramp, and as the tissue compacts over time, the passive tension exerted by the tissue would move the tissue up the ramp towards the final intended position on the upright posts. The trough shape is designed for the tissue seeding mixture to form into a tissue strip after undergoing compaction. Trough dimensions surrounding the ramps can be altered to finely control final tissue dimensions, such as tissue thickness.

Design Number 2 features two upright posts for tissue attachment with no ramp. In some embodiments or iterations of this Design, a trough may be used to concentrate the tissue seeding mixture around the posts.

The posts may include a feature at the tip of the post for tissue retention as described in paragraph [0049].

Design Number 3 features two posts inserted from above and downwards into a 96-well plate. In some embodiments, the design is adapted to other multi-well formats, such as 48-well, 24-well, 12-well, 6-well formats, and the like. The design includes access gaps to allow for media and other substances to be freely added or removed from the well, and for electrodes to be freely inserted from above for electrical stimulation.

Design Number 4 and Design Number 5 are both based on using thin elastomer (e.g., silicone) sheets as the main attachment material. The thin silicone sheet can be formed in a variety of geometries for the tissue to anchor upon, such as by cutting a silicone sheet to the desired size and/or shape. The thin silicone can then be stacked with other materials to fabricate the final culture plate. Design Number 4 utilizes a vertical stacking method in which the tissue anchors are attached to the plate in a horizontal orientation and attached to the bottom of a 96-well plate. Design Number 5 utilizes a horizontal stacking method in which the combined layers are inserted from above into a 96-well plate, similar to Design Number 3, but allowing the force sensor fabrication by laser cutting rather than soft lithographic casting. In some embodiments, the attachment material is made of, but not limited to, other elastomers (such as polyurethane, polyethylene, and the like) or flexible materials.

*Fabrication and Assembly of Curved Post Culture Plates*

The designs of the 96-well inserts with curved upright posts (Figure 2A) were produced using AutoCAD (Autodesk), and fabricated in a 2-step casting process (Figure 2B). The design includes a central tissue seeding area connected to two electrode retaining areas that permit electrical field stimulation of the tissue (Figure 2C). The channels that connect the tissue seeding area to the electrode area are designed such that surface tension of the tissue seeding mixture prevents leakage of the mixture out of the seeding area (Figure 2D). Positive master molds were 3D-printed (ProJet 6000HD, 3D Systems, or Form 3, Formlabs) using stereolithography (SLA) to create a 4x4 array of inserts. In some embodiments, insert size is increased (e.g., 4x8 or 8x12 array). The master molds were silanized using trichloro(1H, 1H,2H,2H-perfluorooctyl)silane (PFOCTS) before use to prevent adhesion and facilitate separation after casting. Polydimethylsiloxane (PDMS, Sylgard 184, Dow Corning) was mixed at 10:1 (base:curing agent) and degassed to remove trapped air bubbles. In some embodiments, the PDMS mixing ratio of base:curing agent is altered (*e.g.*, 5:1 to 20:1) to adjust material stiffness. Using aluminum foil enclosures, 25 g of PDMS was cast over each master mold and further degassed. The PDMS was cured for 48 hours at room temperature followed by 2 hours at 65°C. The resulting PDMS negative molds were silanized using PFOCTS, then cast with 7 g of PDMS to form positive inserts for the 96-well plate. After degassing, the PDMS was cured for 2 hours at 65°C. The PDMS inserts were sealed to the base of a polystyrene bottomless 96-well plate (Greiner Bio-One) using adhesives (Figure 2E) such as silicone adhesive (Silastic Medical Adhesive Silicone, Type A), cyanoacrylate (Loctite 435) or medical tape (double-sided 3M Microfluidic Medical Tape).

## *Example 2*

### Force Measurement of Tissues on Curved Posts

Brightfield microscopy was used to image the post movements over time due to beating of the attached tissue strips (Figure 3A). Tracking and recording of the post positions were performed using a custom LabVIEW code (Figure 4). The position data were analyzed using a custom MATLAB code using a beam-bending equation to estimate the applied forces (Figure 4) [9]. That beam-bending equation is $F = \frac{3\pi E R^4}{2a^2(3L-a)}\delta$ , where F is force, E is Young's modulus of the post material, R is radius, a is height at which force is applied on the post, L is full post height, and delta is the displacement of the center of the post. Tissues were estimated to anchor at the center of the curved portion of the post. The system was engineered and intended to focus tissue attachment at the center of the curved portion and then visually confirmed afterwards using live tissue cultures. Finite element modeling was performed to confirm whether classical beam theory could be used to approximate the force of the cardiac tissues attached to curved posts (Figure 3C). Curved posts were found to displace with approximately 0.5% difference than an equivalent straight post. Because force is linearly proportional to tip deflection for a point-loaded cantilever beam, this translates to <0.5% error in the calculated twitch force, which was considered acceptable.

## *Example 3*

### Fabrication and Assembly of Vertical Stacked Cardiac Tissue Culture Plates

To fabricate the planar cardiac tissue prototype plate, a stacking or layering approach was undertaken (Figure 6), which consists of the following steps: 1) bond thin silicone sheet to commercial bottomless polystyrene cell culture plates, 2) laser cut thin silicone sheet in desired pattern, 3) fabricate trough layer for tissue formation, and 4) adhere trough layer to bottomless plate.

## *Example 4*

### Bonding Thin Silicone Sheet to Commercial Bottomless Polystyrene Cell Culture Plates

Thin silicone sheets (50 to 300 $\mu$m thickness, Elastosil, Wacker Chemie AG) were covalently bonded to bottomless 96-well polystyrene plates through the use of organosilanes [10]. Briefly, the bottomless polystyrene plates were placed into a mixture bath that consisted of 2% (3-Mercaptopropyl)trimethoxysilane and 98% methanol for 1.5 minutes. The polystyrene plates were submerged in water to wash and rid of any excess silane that did not attach to the surface. Both the plate and sheet were than exposed to oxygen plasma at 300 mTorr for 3 minutes. Immediately after the plasma treatment, the sheet was aligned and adhered to the base of the bottomless plate. To strengthen the bond, the plate was placed into a 60°C oven for 2 hours or more. In some embodiments, a thicker silicone sheet may be used to reduce displacement of anchors for tissues that exert larger forces, while a thinner silicone sheet may be used to increase the displacement of the anchors for a given force.

## *Example 5*

### Laser-cutting Thin Silicone Sheet

To fabricate the features of the arms from a silicone sheet, a 30-Watt $CO_2$ laser cutter was utilized (Figure 7A). In some embodiments, the silicone features may be cut using alternative methods, such as a die-cutting machine or vinyl cutter. The silicone arms include a feature to which the cardiac tissue attaches after compaction. As the tissue contracts and exerts force inwards, the silicone arms stretch inwards and the distance of stretch is measured (Figure 7B). The silicone arms can be varied in silicone sheet thickness and arm width to control the amount of displacement. With each design, the conversion from displacement to force can be simply approximated using the stress-strain relationship of the silicone material. To confirm the results, finite element modeling was performed to help determine an optimal design for biological experiments (Figure 7C).

## *Example 6*

### Trough Layer Fabrication

The trough layer provides an area that confines the cell seeding solution around both arms by exploiting the hydrophobic surface properties of the trough material, and the surface tension of the cell seeding solution. In some embodiments, the trough is designed with a circular pedestal such that the seeding mixture sits on top of the pedestal, which was designed such that the seeding cell mixture would form a hemisphere where the arms would be positioned (Figure 8A). In some embodiments, the trough was designed as a circular recessed well such that the seeding cell mixture would reside within (Figure 8B). An acrylic mold of a 96-well circular trough array was fabricated with a laser cutter (Figure 8D). A 2-step cast process (similar to the Curved Post casting described in Example 1) was then performed with a PDMS mixture (10:1 base:crosslinker) to form the trough (Figure 8E). An adhesive layer was placed on top of the PDMS trough layer. Using a laser cutter, material over the wells was removed (Figure 8G). For final assembly, a plasma-free approach was undertaken in which the adhesive layer and the PDMS trough layer were pressed onto the base of the polystyrene plate and baked at 60°C for 48 hours to provide a permanent seal. This seal was leak-resistant to $H_2O$, ethanol, and methanol.

In some embodiments, the trough was designed as a PDMS insert similar to that of the curved post insert described earlier. This cell seeding trough was designed in a rectangular shape with openings for the thin silicone arms to access the tissue area and an electrode area to permit electrical field stimulation of the tissue. The PDMS insert was double-casted from a 3D-printed insert (Figure 9), similarly to previously mentioned methods, and sealed to the plate using an adhesive, such as cyanoacrylate (Loctite 435). In some embodiments, the trough is fabricated from other cell culture-compatible materials, such as thermoformable polystyrene.

## References

1. JL Stevens, TK Baker. (2009). The future of drug safety testing: expanding the view and narrowing the focus. *Drug Discovery Today*. 14. 162-167.
2. F De Clerck, et al. (2002). In vivo measurement of QT prolongation, dispersion and arrhythmogenesis: application to the preclinical cardiovascular safety pharmacology of a new chemical entity. Fundamental & Clinical Pharmacology. 16. 125-140.
3. T Magdy, et al. (2018). Human Induced Pluripotent Stem Cell (hiPSC)-Derived Cells to Assess Drug Cardiotoxicity: Opportunities and Problems. Annual Review of Pharmacology and Toxicology. 58. 83-103.
4. Gintant, G., Sager, P. T., Stockbridge, N. (2016). Evolution of strategies to improve preclinical cardiac safety testing. Nature Reviews Drug Discovery, 15(7), 457-471.
5. Passier, R., Orlova, V., Mummery, C. (2016). Complex Tissue and Disease Modeling using hiPSCs. Cell Stem Cell, 18(3), 309-321.
6. Lee, E. K., et al. (2017). Machine Learning of Human Pluripotent Stem Cell-Derived Engineered Cardiac Tissue Contractility for Automated Drug Classification. Stem Cell Reports, 9(5), 1560-1572.
7. Hughes, JP, et al. (2011). Principles of early drug discovery. British Journal of Pharmacology. 162(6). 1239-1249.
8. Weng, Zhihui, et al. "A simple, cost-effective but highly efficient system for deriving ventricular cardiomyocytes from human pluripotent stem cells." Stem cells and development. (2014): 1704-1716.
9. Serrao, Gregory W., et al. "Myocyte-depleted engineered cardiac tissues support therapeutic potential of mesenchymal stem cells." Tissue Engineering Part A 18.13-14 (2012): 1322-1333.
10. Phan, Duc TT, et al. "A vascularized and perfused organ-on-a-chip platform for large-scale drug screening applications." Lab on a Chip 17.3 (2017): 511-520.

## Claims

1. A microfabricated device comprising an intact and functional miniature tissue for high-throughput screening of modulators of biological tissue activity comprising at least two biocompatible posts and a substrate to which the posts are attached, wherein the distance separating at least two posts is at least 0.5 mm, and wherein each post comprises an elastomeric material, a force sensor, and a feature for tethering the intact and functional miniature tissue to form into a tissue strip, wherein the tissue strip comprises a composition comprising cells of at least one force-generating cell type and one type of extracellular matrix, further wherein the microfabricated device is suitable for monitoring tissue strip position over time, *in situ,*
**characterized in that** the feature for tethering a tissue strip is a curve in the post.

2. The microfabricated device of claim 1, wherein the suitability for monitoring tissue strip position over time is further suitable for providing a measurement of tissue strip movement *in situ.*

3. A system for measuring tissue activity comprising:

   (a) a microfabricated device of claim 1; and
   (b) a recording device for capturing the force detected by at least one force sensor of the microfabricated device.

4. A method for assaying a property of a tissue strip comprising:

   (a) exposing the tissue strip in a microfabricated device of claim 1 to an electrical stimulus or a modulator of a biological activity; and
   (b) measuring the response of the tissue strip, wherein the response of the tissue strip is compared to a baseline measurement of a tissue strip of the same cell type or types not exposed to the electrical stimulus or modulator of a biological activity.

5. The microfabricated device of claim 1, the system of claim 3 or the method of claim 4, wherein the force-generating cell type is a smooth muscle cell type, a skeletal muscle cell type, a cardiac muscle cell type, a fibroblast cell type, a neutrophil cell type, an eosinophil cell type, a basophil cell type, a monocyte cell type, a lymphocyte cell type, a glial cell type, a chondrocyte cell type, an osteoblast cell type, an osteoclast cell type, an osteocyte cell type, a keratinocyte cell type, a melanocyte cell type, a Merkel cell type, a dendritic cell type, an endothelial cell type, an epithelial cell type, a white adipocyte cell type, a brown adipocyte cell type, an esophageal cell type, a pharynx cell type, a larynx cell type, a lung cell type, an hepatocyte cell type, a bladder cell type, a kidney cell type, a stomach cell type, a gallbladder cell type, beta islet cell type, a spleen cell type, a small intestine cell type, or a colon cell type.

6. The microfabricated device of claim 1, the system of claim 3 or the method of claim 4, wherein the force-generating cell type is a stem cell-derived cell type or a myocyte cell type.

7. The microfabricated device of claim 6, the system of claim 6 or the method of claim 6, wherein the myocyte is a cardiomyocyte, preferably a human cardiomyocyte.

8. The microfabricated device of claim 7, the system of claim 7 or the method of claim 7, wherein the human cardiomyocyte is a human ventricular cardiomyocyte.

9. The microfabricated device of claim 1, wherein the elastomeric material is polyurethane, polyethylene, polyacrylamide, or silicone, wherein the silicone preferably is polydimethylsiloxane.

10. The microfabricated device of claim 1 wherein the substrate is thin-layer polyurethane, thin-layer polyethylene, thin-layer polyacrylamide or thin-layer silicone, wherein the thin-layer silicone preferably is thin-layer polydimethylsiloxane.

11. The microfabricated device of claim 1, further comprising a recording device to monitor tissue strip position or to detect movement of a tissue strip.

12. The microfabricated device of claim 1, further comprising two unipolar electrodes for field stimulation, a bipolar micro-electrode for point contact stimulation, or a micro-cannula for contacting a tissue strip with an electrical stimulus or a modulator of a biological tissue activity.

13. The system of claim 3, further comprising two unipolar electrodes for field stimulation, a bipolar micro-electrode for point contact stimulation, or a micro-cannula for contacting a tissue composition comprising the cells of at least one cell type with an electrical stimulus or a modulator of a biological tissue activity.

14. The method of claim 4, wherein movement of the tissue strip results from a change in contractile force.

**Patentansprüche**

1. Eine mikrofabrizierte Vorrichtung, die ein intaktes und funktionelles Miniaturgewebe für das Hochdurchsatz-Screening von Modulatoren der biologischen Gewebeaktivität umfasst, die mindestens zwei biokompatible Stifte und ein Substrat umfasst, an dem die Stifte befestigt sind, wobei der Abstand zwischen mindestens zwei Stiften mindestens 0,5 mm beträgt und wobei jeder Stift ein elastomeres Material, einen Kraftsensor und eine Funktion zum Binden des intakten und funktionellen Miniaturgewebes umfasst, um einen Gewebestreifen zu formen, wobei der Gewebestreifen eine Zusammensetzung umfasst, die Zellen von mindestens einem krafterzeugenden Zelltyp und einen Typ von extrazellulärer Matrix umfasst,

wobei ferner die mikrofabrizierte Vorrichtung dazu geeignet ist, dass die Position des Gewebestreifens über die Zeit *in situ* überwacht werden kann,
**dadurch gekennzeichnet, dass** das Merkmal zur Befestigung eines Gewebestreifens eine Kurve im Stift ist.

2. Die mikrofabrizierte Vorrichtung nach Anspruch 1, wobei die Eignung zur Überwachung der Position des Gewebestreifens über die Zeit ferner geeignet ist, eine Messung der Bewegung des Gewebestreifens *in situ* zu ermöglichen.

3. Ein System zur Messung der Gewebeaktivität, bestehend aus:

(a) einer mikrofabrizierten Vorrichtung nach Anspruch 1; und
(b) einer Aufzeichnungsvorrichtung zum Erfassen der von mindestens einem Kraftsensor der mikrofabrizierten Vorrichtung erfassten Kraft.

4. Ein Verfahren zur Bestimmung einer Eigenschaft eines Gewebestreifens, das Folgendes umfasst:

(a) Aussetzen des Gewebestreifens in einer mikrofabrizierten Vorrichtung nach Anspruch 1 einem elektrischen Stimulus oder einem Modulator einer biologischen Aktivität; und

(b) Messen der Reaktion des Gewebestreifens, wobei die Reaktion des Gewebestreifens mit einer Basislinienmessung eines Gewebestreifens desselben Zelltyps oder derselben Zelltypen verglichen wird, der nicht dem elektrischen Stimulus oder Modulator einer biologischen Aktivität ausgesetzt war.

5. Die mikrofabrizierte Vorrichtung nach Anspruch 1, das System nach Anspruch 3 oder das Verfahren nach Anspruch 4, wobei der krafterzeugende Zelltyp ein glatter Muskelzelltyp, ein Skelettmuskelzelltyp, ein Herzmuskelzelltyp, ein Fibroblastenzelltyp, ein Neutrophilen-Zelltyp, ein Eosinophilen-Zelltyp, ein Basophilen-Zelltyp, ein Monozyten-Zelltyp, ein Lymphozyten-Zelltyp, ein Glia-Zelltyp, ein Chondrozyten-Zelltyp, ein Osteoblasten-Zelltyp, ein Osteoklasten-Zelltyp, ein Osteozyten-Zelltyp, ein Keratinozyten-Zelltyp, ein Melanozyten-Zelltyp, ein Merkel-Zelltyp, ein dendritischer Zelltyp, ein Endothel-Zelltyp, ein Epithel-Zelltyp, ein weißer Adipozyten-Zelltyp, ein brauner Adipozyten-Zelltyp, ein Ösophagus-Zelltyp, ein Rachen-Zelltyp, ein Kehlkopfzelltyp, ein Lungenzelltyp, ein Hepatozytenzelltyp, ein Blasenzelltyp, ein Nierenzelltyp, ein Magenzelltyp, ein Gallenblasenzelltyp, ein Beta-Inselzelltyp, ein Milzzelltyp, ein Dünndarmzelltyp oder ein Dickdarmzelltyp ist.

6. Die mikrofabrizierte Vorrichtung nach Anspruch 1, das System nach Anspruch 3 oder das Verfahren nach Anspruch 4, wobei der Kraft erzeugende Zelltyp ein von Stammzellen abgeleiteter Zelltyp oder ein Myozyten-Zelltyp ist.

7. Die mikrofabrizierte Vorrichtung nach Anspruch 6, das System nach Anspruch 6 oder das Verfahren nach Anspruch 6, wobei der Myozyt ein Kardiomyozyt, vorzugsweise ein menschlicher Kardiomyozyt ist.

8. Die mikrofabrizierte Vorrichtung nach Anspruch 7, das System nach Anspruch 7 oder das Verfahren nach Anspruch 7, wobei der menschliche Kardiomyozyt ein menschlicher ventrikulärer Kardiomyozyt ist.

9. Die mikrofabrizierte Vorrichtung nach Anspruch 1, wobei das elastomere Material Polyurethan, Polyethylen, Polyacrylamid oder Silikon ist, wobei das Silikon vorzugsweise Polydimethylsiloxan ist.

10. Die mikrofabrizierte Vorrichtung nach Anspruch 1, wobei das Substrat ein dünnschichtiges Polyurethan, dünnschichtiges Polyethylen, dünnschichtiges Polyacrylamid oder dünnschichtiges Silikon ist, wobei das dünnschichtiges Silikon vorzugsweise ein dünnschichtiges Polydimethylsiloxan ist.

11. Die mikrofabrizierte Vorrichtung nach Anspruch 1, umfassend ferner eine Aufzeichnungsvorrichtung zur Überwachung der Position des Gewebestreifens oder zur Erfassung der Bewegung eines Gewebestreifens.

12. Die mikrofabrizierte Vorrichtung nach Anspruch 1, umfassend ferner zwei unipolare Elektroden für die Feldstimulation, eine bipolare Mikroelektrode für die Punktkontaktstimulation oder eine Mikrokanüle für den Kontakt eines Gewebestreifens mit einem elektrischen Stimulus oder einem Modulator einer biologischen Gewebeaktivität.

13. Das System nach Anspruch 3, das ferner zwei unipolare Elektroden für die Feldstimulation, eine bipolare Mikroelektrode für die Punktkontaktstimulation oder eine Mikrokanüle für den Kontakt einer Gewebezusammensetzung, die die Zellen mindestens eines Zelltyps umfasst, mit einem elektrischen Stimulus oder einem Modulator einer biologischen Gewebeaktivität umfasst.

14. Das Verfahren nach Anspruch 4, wobei die Bewegung des Gewebestreifens aus einer Änderung der kontraktilen Kraft resultiert.

## Revendications

1. Dispositif microfabriqué comprenant un tissu miniature intact et fonctionnel pour le criblage à haut débit de modulateurs de l'activité des tissus biologiques, comprenant au moins deux poteaux biocompatibles et un substrat auquel les poteaux sont fixés, dans lequel la distance séparant au moins deux poteaux est d'au moins 0.5 mm, et dans lequel chaque poteau comprend un matériau élastomère, un capteur de force et une caractéristique permettant d'attacher le tissu miniature intact et fonctionnel pour former une bande de tissu, dans lequel la bande de tissu comprend une composition comprenant des cellules d'au moins un type de cellule génératrice de force et un type de matrice extracellulaire, et dans lequel le dispositif microfabriqué permet de surveiller la position de la bande de tissu au fil du temps, *in situ,* **caractérisé par le fait que** l'élément permettant d'attacher une bande de tissu est une courbe dans le poteau.

2. Dispositif microfabriqué selon la revendication 1, dans lequel l'aptitude à surveiller la position de la bande de tissu dans le temps est également conçue pour fournir une mesure du mouvement de la bande de tissu *in situ.*

3. Système de mesure de l'activité tissulaire comprenant

    (a) un dispositif microfabriqué selon la revendication 1 ; et
    (b) un dispositif d'enregistrement pour capturer la force détectée par au moins un capteur de force du dispositif microfabriqué.

4. Procédé d'évaluation d'une propriété d'une bande de tissu comprenant les opérations suivantes :

    (a) exposer la bande de tissu dans un dispositif microfabriqué selon la revendication 1 à un stimulus électrique ou à un modulateur d'une activité biologique ; et
    (b) mesurer la réponse de la bande de tissu, la réponse de la bande de tissu étant comparée à une mesure de référence d'une bande de tissu du ou des mêmes types de cellules non exposées au stimulus électrique ou au modulateur d'une activité biologique.

5. Dispositif microfabriqué selon la revendication 1, système selon la revendication 3 ou procédé selon la revendication 4, dans lequel le type de cellule génératrice de force est un type de cellule musculaire lisse, un type de cellule musculaire squelettique, un type de cellule musculaire cardiaque, un type de cellule fibroblaste, un type de cellule neutrophile, un type de cellule éosinophile, un type de cellule basophile, un type de cellule monocytaire, un type de cellule lymphocytaire, un type de cellule gliale, un type de cellule chondrocyte, un type de cellule ostéoblaste, un type de cellule ostéoclaste, un type de cellule ostéocyte, un type de cellule kératinocytaire, un type de cellule mélanocytaire, un type de cellule de Merkel, un type de cellule dendritique, un type de cellule endothéliale, un type de cellule épithéliale, un type de cellule adipocytaire blanche, un type de cellule adipocytaire brune, un type de cellule oesophagienne, un type de cellule du pharynx, un type de cellule du larynx, un type de cellule du poumon, un type de cellule de l'hépatocyte, un type de cellule de la vessie, un type de cellule du rein, un type de cellule de l'estomac, un type de cellule de la vésicule biliaire, un type de cellule des îlots de Langerhans, un type de cellule de la rate, un type de cellule de l'intestin grêle ou un type de cellule du côlon.

6. Dispositif microfabriqué selon la revendication 1, système selon la revendication 3 ou procédé selon la revendication 4, dans lequel le type de cellule génératrice de force est un type de cellule dérivé d'une cellule souche ou un type de cellule myocytaire.

7. Dispositif microfabriqué selon la revendication 6, système selon la revendication 6 ou procédé selon la revendication 6, dans lequel le myocyte est un cardiomyocyte, de préférence un cardiomyocyte humain.

8. Dispositif microfabriqué selon la revendication 7, système selon la revendication 7 ou procédé selon la revendication 7, dans lequel le cardiomyocyte humain est un cardiomyocyte ventriculaire humain.

9. Dispositif microfabriqué selon la revendication 1, dans lequel le matériau élastomère est du polyuréthane, du polyéthylène, du polyacrylamide ou du silicone, le silicone étant de préférence du polydiméthylsiloxane.

10. Dispositif microfabriqué selon la revendication 1, dans lequel le substrat est un polyuréthane en couche mince, un polyéthylène en couche mince, un polyacrylamide en couche mince ou un silicone en couche mince, le silicone en couche mince étant de préférence un polydiméthylsiloxane en couche mince.

11. Dispositif microfabriqué selon la revendication 1 comprend en outre un dispositif d'enregistrement pour surveiller la position de la bande de tissu ou pour détecter le mouvement d'une bande de tissu.

12. Dispositif microfabriqué selon la revendication 1 comprend en outre deux électrodes unipolaires pour la stimulation de champ, une microélectrode bipolaire pour la stimulation par contact ponctuel, ou une microcanule pour la mise en contact d'une bande de tissu avec un stimulus électrique ou un modulateur d'une activité tissulaire biologique.

13. Système selon la revendication 3 comprend en outre deux électrodes unipolaires pour la stimulation de champ, une microélectrode bipolaire pour la stimulation par contact ponctuel, ou une microcanule pour mettre en contact une composition tissulaire comprenant les cellules d'au moins un type cellulaire avec un stimulus électrique ou un modulateur d'une activité tissulaire biologique.

14. Procédé selon la revendication 4, dans lequel le mouvement de la bande de tissu résulte d'une modification de la force contractile.

| #1: Upright Posts Ramp | #2: Upright Posts No Ramp | #3: Inverted Posts | #4: Vertical Stacked Silicone Sheet | #5: Horizontal Stacked Silicone Sheet |
|---|---|---|---|---|
| | | | | |

Figure 1

**A)**

**B)** 2-Step Casting Process

PDMS

Casting Mold

PDMS

PDMS Casting Mold

**C)**

**D)**

**E)**

Figure 2

Figure 3

Figure 4

Figure 5

Figure 6

A)

B)

Force        Force

Cardiac
Tissue

C)

0.8167 micron

B.C.:
@ r = Well Diameter
ΔX = 0

1.25

0 Min.

Figure 7

Figure 8

Figure 9

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2014085933 A

## Non-patent literature cited in the description

- **ARNE HANSEN et al.** Development of a drug screening platform based on engineered heart tissue. *CIRCULATION RESEARCH, GRUNE AND STRATTON, BALTIMORE, US,* 09 July 2010, vol. 107 (1), ISSN 0009-7330, 35
- **INGRA VOLLERT et al.** In-vitro perfusion of engineered heart tissue through endothelialized channels. *TISSUE ENGINEERING PART A,* 25 October 2013, ISSN 1937-3341
- the Merck Manual of Diagnosis and Therapy. Merck Research Laboratories, 2006
- The Encyclopedia of Molecular Biology. Blackwell Science Ltd, 1994
- **CROWTHER J. R.** the ELISA Guidebook (Methods in Molecular Biology. 2000, 149
- Definitions of common terms in molecular biology. **BENJAMIN LEWIN.** Genes X. Jones & Bartlett Publishing, 2009
- Biology and Biotechnology: A Comprehensive Desk Reference. VCH Publishers, Inc, 1995
- **SAMBROOK et al.** Molecular Cloning: A Laboratory Manual. Cold Spring Harbor Laboratory Press, 2001
- **DAVIS et al.** Basic Methods in Molecular Biology. Elsevier Science Publishing, Inc, 1995
- Current Protocols in Cell Biology (CPCB). John Wiley and Sons, Inc
- **R. IAN FRESHNEY.** Culture of Animal Cells: A Manual of Basic Technique. Wiley-Liss, 2005
- Animal Cell Culture Methods. Methods in Cell Biology. academic press, 1998, vol. 57
- **TAKAHASHI et al.** *Cell,* 2006, vol. 126, 663-676
- **TAKAHASHI et al.** *Cell,* 2007, vol. 131, 861-872
- **NAKAGAWA et al.** *Nat. Biotechnol.,* 2008, vol. 26, 101-106
- **F DE CLERCK et al.** In vivo measurement of QT prolongation, dispersion and arrhythmogenesis: application to the preclinical cardiovascular safety pharmacology of a new chemical entity. *Fundamental & Clinical Pharmacology,* 2002, vol. 16, 125-140
- **T MAGDY et al.** Human Induced Pluripotent Stem Cell (hiPSC)-Derived Cells to Assess Drug Cardiotoxicity: Opportunities and Problems. *Annual Review of Pharmacology and Toxicology,* 2018, vol. 58, 83-103
- **GINTANT, G. ; SAGER, P. T. ; STOCKBRIDGE, N.** Evolution of strategies to improve preclinical cardiac safety testing. *Nature Reviews Drug Discovery,* 2016, vol. 15 (7), 457-471
- **PASSIER, R. ; ORLOVA, V. ; MUMMERY, C.** Complex Tissue and Disease Modeling using hiPSCs. *Cell Stem Cell,* 2016, vol. 18 (3), 309-321
- **LEE, E. K. et al.** Machine Learning of Human Pluripotent Stem Cell-Derived Engineered Cardiac Tissue Contractility for Automated Drug Classification. *Stem Cell Reports,* 2017, vol. 9 (5), 1560-1572
- **HUGHES, JP et al.** Principles of early drug discovery. *British Journal of Pharmacology,* 2011, vol. 162 (6), 1239-1249
- **WENG, ZHIHUI et al.** A simple, cost-effective but highly efficient system for deriving ventricular cardiomyocytes from human pluripotent stem cells. *Stem cells and development,* 2014, 1704-1716
- **SERRAO, GREGORY W. et al.** Myocyte-depleted engineered cardiac tissues support therapeutic potential of mesenchymal stem cells. *Tissue Engineering Part,* 2012, vol. 18 (13-14), 1322-1333
- **PHAN, DUC TT et al.** A vascularized and perfused organ-on-a-chip platform for large-scale drug screening applications. *Lab on a Chip,* 2017, vol. 17 (3), 511-520